Europäisches Patentamt

European Patent Office

Office européen des brevets

(19)

(11) Numéro de publication: **0 247 980 B1**

(12) **FASCICULE DE BREVET EUROPEEN**

(45) Date de publication de fascicule du brevet:
14.08.91

(21) Numéro de dépôt: **87830202.5**

(22) Date de dépôt: **29.05.87**

(51) Int. Cl.⁵: **A61K 33/26**, A61K 31/295,
A61K 9/00

(54) Presentation pharmaceutique pour l'administration orale de sels ferreux utilisables en thérapie dans les formes d'anémie avec carence de fer.

(30) Priorité: **30.05.86 IT 940786**

(43) Date de publication de la demande:
**02.12.87 Bulletin 87/49**

(45) Mention de la délivrance du brevet:
**14.08.91 Bulletin 91/33**

(84) Etats contractants désignés:
**AT CH DE ES FR GB GR LI NL SE**

(56) Documents cités:
**RO-A- 0 076 690**

CHEMICAL ABSTRACTS, vol. 100, no. 1, 2 janvier 1984, Columbus, Ohio, US, abstract no. 12648p; S.CHERECHES et al.: "Unit-dose product for treating anemie in infants" & RO-A-76690

CHEMICAL ABSTRACTS, vol. 103, no. 25, 23 décembre 1985, Columbus, Ohio, US, abstract no. 220839r; A. BOTA: "Antianemic syrup" & RO-A-86113

CHEMICAL ABSTRACTS, vol. 93, no. 10, 8

septembre 1980, Columbus, Ohio, US, abstract no. 101441v; V. BOTA et al.: "Study on some syrups prepared form fruits and iron-enriched plant extracts"

(73) Titulaire: **A. MENARINI INDUSTRIE FARMA-CEUTICHE RIUNITE S.R.L.**
**Via Sette Santi 3**
**I-50131 Firenze(IT)**

(72) Inventeur: **Bani, Raffaello**
**Via di Chiana No. 56**
**I-50127 Firenze(IT)**
Inventeur: **Ghelardoni, Mario**
**Via Lambruschini No. 5**
**I-50134 Firenze(IT)**
Inventeur: **Melani, Francesco**
**Via Poggio Magherini No. 5**
**I-50014 Fiesole Firenze(IT)**

(74) Mandataire: **Mannucci, Gianfranco, Dott.-Ing.**
**Ufficio Tecnico Ing. A. Mannucci Via della**
**Scala 4**
**I-50123 Firenze(IT)**

## Description

L'invention concerne une présentation pharmaceutique en sirop, constituée de petits flacons contenant un support édulcoré et aromatisé, munis de bouchons de récipient hermétiquement fermés contenant le principe actif qui est constitué par un sel ferreux utilisable en thérapie, tel que sulfate, aspartate, fumarate, citrate, succinate, tartrate et, notamment, gluconate ou autres. Au moment de l'utilisation, le contenu du bouchon du récipient est trsnsféré, dans le support édulcoré et aromatisé par une opération immédiate.

Il convient de noter que les ions ferreux administrés par voie orale sont plus facilement absorbés que les ions ferriques, mais que la présentation pharmaceutique pour l'administration doit être conçue pour limiter au maximum l'oxydation du principe actif.

Il convient de remarquer également que les flacons classiques de sirop contenant un sel ferreux en solution ne constituent pas la présentation pharmaceutique la plus adéquate pour ledit principe actif, dans la mesure où l'oxydation des ions ferreux en ions ferriques augmente fortement au fur et à mesure que le niveau du sirop diminue dans le flacon au cours de l'utilisation thérapeutique et que le volume d'air augmente donc au-dessus du sirop. Enfin, il convient de remarquer que les sirops à faible concentration de sels ferreux subissent l'oxydation, même dans les conteneurs monodose comportant une fermeture classique, par suite de la présence inévitable d'oxygène au cours de la fabrication.

Grâce à l'utilisation de récipients monodose munis de bouchons de récipients hermétiquement fermés, dans lesquels est contenu le sel ferreux et, plus particulièrement, le gluconate ferreux sous forme solide, il est possible d'assurer, selon la présente invention, les meilleures conditions pour garantir une amélioration de la stabilité des ions ferreux vis-à-vis de l'oxydation.

Le Brevet RO-A-76690 (Chemical Abstracts 100:12648p - 1984) concerne des unidoses contenant un sel ferreux, sous forme d'une solution contenant des additifs visés à inhiber l'oxydation en sel ferrique. La réalisation de telles solutions est difficile, sur le plan d'une production industrielle, pour laquelle est très difficile d'établir une atmosphère exempte d'oxygène. La difficulté devient encore majeure si on doit prévoir des unidoses, et encore plus si on doit employer des récipients en verre - ampoules qui doivent être fermées par moulage et soudure à la flamme. Des récipients en matières plastiques ne sont pas acceptables, à cause de leur perméabilité aux gaz. La production est compliquée, les risques d'une présence d'oxygène sont très élevés et des contrôles ne sont pas fiables. Les produits peuvent être inactifs ou devenir inactifs après quelques temps.

En remplaçant la solution stabilisée de sel ferreux par du sel ferreux stabilisé par son seul état solide selon l'invention, on obtient un produit qui n'a pas de problèmes de modification par oxygenation, même après beaucoup de temps et même si le récipient unidose est en matière plastique. La production est très simplifiée, n'ayant pas besoin de travailler en atmosphère d'azote.

Un produit unidose en verre (qui doit être une ampoule soudée pour assurer la conservation sans oxydation) d'une solution contenant le sel ferreux doit être brisée, avec une manipulation ni rapide ni sûre, et bien plus compliquée que celle d'un produit en matière plastique avec le compartiment pour le sel.

L'invention concerne également une formulation de la présentation pharmaceutique particulièrement adéquate pour l'application décrite ci-dessus.

Le sirop contenu dans le petit flacon peut être constitué d'agents édulcorants appropriés tels que saccharose, fructose, sorbitose, saccharine, cyclamate de sodium, aspartame et d'autres encore, d'agents aromatisants appropriés tels que les arômes de fruits, de menthe et autres encore, d'agents de conservation appropriés tels que le p-hydroxybenzoate de méthyle et de propyle, l'acide sorbique, l'alcool éthylique, la glycérine, et d'autres encore, d'anti-oxydants appropriés tels que l'acide ascorbique, le métabisulfite, le formaldéhyde, le sulfoxylate sodique et d'autres encore. Le support peut être constitué d'eau purifiée contenant un acide organique ou minéral permettant d'obtenir un pH compris entre 3 et 6. La poudre contenue dans le bouchon du récipient peut être constituée d'un sel ferreux et, en particulier, de gluconate ferreux et de lubrifiants appropriés tels que, par exemple, le stéarate de magnésium, le stéarate de glycérol, l'huile de ricin hydrogénée, la paraffine liquide, le diméticone, et d'autres encore, ainsi que des diluants appropriés.

Les lubrifiants liquides sont préférables, parce qu'ils forment une fine couche sur la surface des cristaux de sels ferreux et les protègent ainsi ultérieurement de l'action oxydante de l'air.

On peut également utiliser comme diluants du principe actif des excipients appropriés tels que le saccharose, le lactose, le glucose, et d'autres encore. A titre d'exemple non limitatif, on décrira les formulations suivantes:

2

EP 0 247 980 B1

### Contenu du bouchon du récipient

- gluconate ferreux: 200-1600 mg et plus particulièrement

300 mg

- diméticone (lubrifiant) 10-80 mg et plus particulièrement

15 mg

### Contenu du petit flacon monodose

| | | | |
|---|---|---|---|
| - fructose | 1000 - 6000 | mg et plus particulièrement | 3000 mg |
| - sorbitose | 300 -2000 | mg et plus particulièrement | 750 mg |
| - glycérol | 300 -2000 | mg et plus particulièrement | 750 mg |
| - méthylparabène | 10 - 20 | mg et plus particulièrement | 15 mg |
| - propylparabène | 2 - 4 | mg et plus particulièrement | 3 mg |
| - arômes de fruits | 10 - 50 | mg et plus particulièrement | 20 mg |
| - acide citrique | q.s.p. pH 3 - 6 | et plus particulièrement | 4,5 |
| - eau purifiée | q.s.p. g 10 - 20 | et plus particulièrement | 15 |

**Revendications**
**Revendications pour les Etats contractants suivants: CH, DE, FR, GB, Li, NL, SE**

**1.** Présentation pharmaceutique pour l'administration orale (sirop), constituée d'un sel de fer sous forme

3

ferreux, utilisable pour la thérapie, caractérisée par le fait d'être utilisée sous forme de petits flacons monodose munis de bouchons de récipient hermétiquement fermés, les flacons contenant un support aqueux édulcoré et aromatisé de manière appropriée et le bouchon du récipient hermétiquement fermé contenant le sel ferreux sous forme solide.

2.  Présentation pharmaceutique selon la revendication 1, caractérisée par le fait que le sel ferreux est du gluconate ferreux additionné de diméticone.

3.  Composition pour usage pharmaceutique à administrer par voie orale (sirop), constituée de gluconate fer̄reux et d'un support approprié en petits flacons monodose, avec un bouchon de récipient contenant le ̄gluconate ferreux à l'état solide.

4.  Produit pharmaceutique avec préparation en flacon monodose et bouchon de récipient selon les revendications précédentes, caractérisé par le fait qu'il possède la formule suivante:

Contenu du bouchon du récipient:

- gluconate ferreux                    mg   200 - 1600

- diméticone                           mg    10 -   80


Contenu du petit flacon monodose:

- fructose                             mg  1000 - 6000

- sorbitose                            mg   300 - 2000

- glycérol                             mg   300 - 2000

- méthylparabène                       mg    10 -   20

- propylparabène                       mg     2 -    4

- aromes de fluits                     mg    10 -   50

- acide citrique        q.s.p.     pH 3-6

- eau purifiée          q.s.p.              g    10 -   20


5.  Produit pharmaceutique selon la revendication 4, caractérisé par le fait qu'il possède la formulation suivante:

<u>Contenu du bouchon du récipient:</u>

| | | |
|---|---|---|
| – gluconate ferreux | mg | 300 |
| – diméticone | mg | 15 |

<u>Contenu du petit flacon monodose:</u>

| | | |
|---|---|---|
| – fructose | mg | 3000 |
| – sorbitose | mg | 750 |
| – glycérol | mg | 750 |
| – méthylparabène | mg | 15 |
| – propylparabène | mg | 3 |
| – arôme de fruit | mg | 20 |
| – acide citrique q.s.p. | pH | 4,5 |
| – eau purifiée q.s.p. | g | 15 |

6. Procédé pour la preparation d'un produit pharmaceutique pour l'administration orale (sirop), constitué d'un sel de fer sous forme ferreux, utilisable pour la thérapie, caractérisé par le fait que l'on confectionne dans des petits flacons monodose un support aqueux édulcoré et aromatisé de manière appropriée et qu'on place le sel ferreux sous dans des bouchons de récipient forme solide hermétiquement fermés.

7. Procédé selon la revendication 6, caractérisé par le fait que le sel ferreux est du gluconate ferreux additionné de diméticone.

8. Procédé selon les revendications 6 et 7, caractérisé par le fait que l'on confectionne dans un bouchon de récipient:

| | | | |
|---|---|---|---|
| – gluconate ferreux | mg | 200 – | 1600 |
| – diméticone | mg | 10 – | 80 |

et dans le petit flacon monodose:

| | | | |
|---|---|---|---|
| – fructose | mg | 1000 – | 6000 |
| – sorbitose | mg | 300 – | 2000 |
| – glycérol | mg | 300 – | 2000 |
| – méthylparabène | mg | 10 – | 20 |

| | | | |
|---|---|---|---|
| – propylparabène | mg | 2 – | 4 |
| – aromes de fruits | mg | 10 – | 50 |
| – acide citrique q.s.p. pH 3–6 | | | |
| – eau purifiée q.s.p. | g | 10 – | 20 |

9. Procédé selon revendication 8 caractérisé par le fait que la formulation suivante est confectionnée dans le bouchon de récipient:

| | | |
|---|---|---|
| – gluconate ferreux | mg | 300 |
| – diméticone | mg | 15 |

et la formulation suivante est confectionnée dans le petit flacon monodose:

| | | |
|---|---|---|
| – fructose | mg | 3000 |
| – sorbitose | mg | 750 |
| – glycérol | mg | 750 |
| – méthylparabène | mg | 15 |
| – propylparabène | mg | 3 |
| – arôme de fruit | mg | 20 |
| – acide citrique  q.s.p.  pH  4,5 | | |
| – eau purifiée  q.s.p.  g  15 | | |

**Revendications pour les Etats contractants suivants: AT, ES, GR**

1.  Procédé pour la preparation d'un produit pharmaceutique pour l'administration orale (sirop), constitué d'un sel de fer sous forme ferreux, utilisable pour la thérapie, caractérisé par le fait que l'on confectionne dans des petits flacons monodose un support aqueux édulcoré et aromatisé de manière appropriée et qu'on place le sel ferreux sous forme solide dans des bouchons de récipient hermétiquement fermés.

2.  Procédé selon la revendication 1, caractérisé par le fait que le sel ferreux est du gluconate ferreux additionné de diméticone.

3.  Procédé selon les revendications 1 et 2, caractérisé par le fait que l'on confectionne dans un bouchon de récipient:

7

| | | | | |
|---|---|---|---|---|
| - gluconate ferreux | mg | 200 | - | 1600 |
| - diméticone | mg | 10 | - | 80 |

et dans le petit flacon monodose:

| | | | | |
|---|---|---|---|---|
| - fructose | mg | 1000 | - | 6000 |
| - sorbitose | mg | 300 | - | 2000 |
| - glycérol | mg | 300 | - | 2000 |
| - méthylparabène | mg | 10 | - | 20 |
| - propylparabène | mg | 2 | - | 4 |
| - aromes de fruits | mg | 10 | - | 50 |

- acide citrique q.s.p.     pH 3-6

| | | | | |
|---|---|---|---|---|
| - eau purifiée    p.s.p. | g | 10 | - | 20 |

4. Procédé selon la revendication 3, caractérisé par le fait que la formulation suivante est confectionnée dans le bouchon de récipient:

EP 0 247 980 B1

| | | |
|---|---|---|
| – gluconate ferreux | mg | 300 |
| – diméticone | mg | 15 |

et la formulation suivante est confectionnée dans le petit flacon monodose:

| | | |
|---|---|---|
| – fructose | mg | 3000 |
| – sorbitose | mg | 750 |
| – glycérol | mg | 750 |
| – méthylparabène | mg | 15 |
| – propylparabène | mg | 3 |
| – arôme de fruit | mg | 20 |
| – acide citrique q.s.p. | pH | 4,5 |
| – eau purifiée q.s.p. | g | 15 |

**Claims**
**Claims for the following Contracting States: CH, DE, FR, GB, Li, NL, SE**

1. A pharmaceutical presentation for oral administration (syrup), comprising an iron salt in the ferrous form, of use for therapy, characterised by being used in the form of small single-dose bottles provided with hermetically sealed receptacle stoppers, the bottles containing a suitably sweetened and flavoured aqueous support and the hermetically sealed receptable stopper containing the ferrous salt in the solid form.

2. A pharmaceutical presentation according to claim 1, characterised in that the ferrous salt is ferrous gluconate to which dimethicone has been added.

3. A composition for pharmaceutical use for oral administration (syrup), comprising ferrous gluconate and a suitable support in small one-dose bottles, with a receptacle stopper containing the ferrous gluconate in the solid state.

4. A pharmaceutical product with a preparation in a one-dose bottle and receptacle stopper according to the preceding claims, characterised in that it has the following formula:

### Contents of receptacle stopper:

| | | |
|---|---|---|
| – ferrous gluconate | mg | 200 - 1600 |
| – dimethicone | mg | 10 - 80 |

### Contents of small one-dose bottle:

| | | |
|---|---|---|
| - fructose | mg | 1000 - 6000 |
| - sorbitose | mg | 300 - 2000 |
| - glycerol | mg | 300 - 2000 |
| - methylparaben | mg | 10 - 20 |
| - propylparaben | mg | 2 - 4 |
| - fruit flavourings | mg | 10 - 50 |
| - citric acid q.s.p. pH 3-6 | | |
| - purified water q.s.p.. | g | 10 - 20 |

5. A pharmaceutical product according to claim 4, characterised in that it has the following formulation:

### Contents of receptacle stopper:

| | | |
|---|---|---|
| - ferrous gluconate | mg | 300 |
| - dimethicone | mg | 15 |

### Contents of small one-dose bottle:

| | | |
|---|---|---|
| - fructose | mg | 3000 |
| - sorbitose | mg | 750 |
| - glycerol | mg | 750 |
| - methylparaben | mg | 15 |
| - propylparaben | mg | 3 |
| - fruit flavourings | mg | 20 |
| - citric acid q.s.p pH 4.5 | | |
| - purified water q.s.p. | g | 15 |

6. A process for the preparation of a pharmaceutical product for oral administration (syrup), comprising an iron salt in the ferrous form, of use for therapy, characterised in that a suitably flavoured and sweetened aqueous support is made in small one-dose bottles and in that the ferrous salt in the solid form is disposed in hermetically scaled receptacle stoppers.

7. A process according to claim 6, characterised in that the ferrous salt is ferrous gluconate to which dimethicone has been added.

8. A process according to claims 6 and 7, characterised in that the following is made in a receptacle stopper:

EP 0 247 980 B1

```
- ferrous gluconate                    mg     200 - 1600
- dimethicone                          mg      10 -   80
```

and in the small one-dose bottle:

```
- fructose                             mg   1000 - 6000
- sorbitose                            mg    300 - 2000
- glycerol                             mg    300 - 2000
- methylparaben                        mg     10 -   20
- propylparaben                        mg      2 -    4
- fruit flavourings                    mg     10 -   50
- citric acid        q.s.p.    pH   3-6
- purified water  q.s.p.               g      10 -   20
```

9. A process according to claim 8, characterised in that the following formulation is made in the receptacle stopper:

```
- ferrous gluconate                    mg     300
- dimethicone                          mg      15
```

and the following formulation is made in the small one-dose bottle:

```
- fructose                             mg   3000
- sorbitose                            mg    750
- glycerol                             mg    750
- methylparaben                        mg     15
- propylparaben                        mg      3
- fruit flavourings                    mg     20
- citric acid        q.s.p. pH 4.5
- purified water  q.s.p    g  15
```

Claims for the following Contracting States: AT, ES, GR

1. A process for the preparation of a pharmaceutical product for oral administration (syrup), comprising an iron salt in the ferrous form, of use for therapy, characterised in that a suitably flavoured and sweetened aqueous support is made in small one-dose bottles and in that the ferrous salt in the solid form is disposed in hermetically sealed receptacle stoppers.

2. A process according to claim 1, characterised in that the ferrous salt is ferrous gluconate to which dimethicone has been added.

11

3. A process according to claims 1 and 2, characterised in that the following is made in a receptacle stopper:

```
- ferrous gluconate              mg    200 - 1600
- dimethicone              .     mg     10 -   80
```

and in the small one-dose bottle:

```
- fructose                  mg  1000 - 6000
- sorbitose                 mg   300 - 2000
- glycerol                  mg   300 - 2000
- methylparaben             mg    10 -   20
- propylparaben             mg     2 -    4
- fruit flavourings         mg    10 -   50
- citric acid    q.s.p.   pH  3-6
- purified water q.s.p.      g    10 -   20
```

4. A process according to claim 3, characterised in that the following formulation is made in the receptacle stopper:

```
- ferrous gluconate              mg    300
- dimethicone                    mg     15
```

and the following formulation is made in the small one-dose bottle:

```
- fructose                  mg  3000
- sorbitose                 mg   750
- glycerol                  mg   750
- methylparaben             mg    15
- propylparaben             mg     3
- fruit flavourings         mg    20
- citric acid    q.s.p. pH 4.5
- purified water q.s.p   g  15
```

**Patentansprüche**
**Patentansprüche für folgende Vertragsstaaten: CH, DE, FR, GB, Li, NL, SE**

1. Arzneimitteldarreichung für die orale Verabreichung (Sirup), bestehend aus einem therapeutisch nutzbaren Eisensalz von zweiwertigem Eisen, dadurch gekennzeichnet, daß sie benutzt wird in Form von kleinen Eindosisfläschchen, die mit luftdicht verschlossenen Behälterstopfen versehen sind, daß die Fläschchen einen wäßrigen Träger enthalten, der in geeigneter weise mit sußstoffen und Aromastoffen versetzt ist, und daß der luftdicht verschlossenen ßehälterstopfen das Eisen (II)-Salz in fester Form enthält.

2. Arzneimitteldarreichung nach Anspruch 1, dadurch gekennzeichnet, daß das Eisen(II)-Salz aus Eisen(II)-Gluconat besteht, dem Dimeticon zugesetzt ist.

3. Zusammensetzung zur pharmazeutischen Verwendung bei oraler Verabreichung (Sirup), bestehend aus Eisen(II)-Gluconat und einem geeigneten Träger In kleinen Eindosisfläschchen, mit einem Behälterstopfen, der das Eisen(II)-Gluconat in fester Form enthält.

4. Arzneimittelprodukt als präparat in Eindosisfläschchen und mit Behälterstopfen, nach den vorhergehenden Ansprüchen, dadurch gekennzeichnet, daß es folgender Rezeptur entspricht:

<u>**Inhalt des Behälterstopfens:**</u>

| | |
|---|---|
| - Eisen(II)-Gluconat | 200 - 1600 mg |
| - Dimeticon | 10 - 80 mg |

<u>**Inhalt des kleinen Eindosisfläschchens:**</u>

| | |
|---|---|
| - Fructose | 1000 - 6000 mg |
| - Sorbitose | 300 - 2000 mg |
| - Glycerin | 300 - 2000 mg |
| - Methylparaben | 10 - 20 mg |
| - Propylparaben | 2 - 4 mg |
| - Fruchtaromen | 10 - 50 mg |
| - Citronensäure | q.s.p. pH 3 - 6 |
| - Reinwasser | q.s.p. 10 - 20 g |

5. Arzneimittelprodukt nach Anspruch 4, dadurch gekennzeichnet, daß es folgendermaßen zusammengesetzt ist:

## Inhalt des Behälterstopfens:

| | |
|---|---|
| - Eisen(II)-Gluconat | 300 mg |
| - Dimeticon | 15 mg |

## Inhalt des kleinen Eindosisfläschchens:

| | |
|---|---|
| - Fructose | 3000 mg |
| - Sorbitose | 750 mg |
| - Glycerin | 750 mg |
| - Methylparaben | 15 mg |
| - Propylparaben | 3 mg |
| - Fruchtaroma | 20 mg |
| - Citronensäure | q.s.p. pH 4,5 |
| - Reinwasser | q.s.p. 15 g |

6. Verfahren zur Herstellung eines Arzneimittelprodukts für die orale Verabreichung (Sirup), bestehend aus einem Eisensalz in Form von therapeutisch nutzbarem zweiwertigem Eisen, dadurch gekennzeichnet, daß in kleinen Eindosisfläschchen ein in geeigneter weise mit Süßstoffen und Aromastoffen versetzter wäßriger Träger zubereitet wird, und daß das Eisen (II)-Salz in fester Form In luftdicht verschlossene Behälterstopfen eingebracht wird.

7. Verfahren nach Anspruch 6, dadurch gekennzeichnet, daß das Eisen(II)-Salz mit Dimeticon angereichertes Eisen(II)-Gluconat ist.

8. Verfahren nach den Ansprüchen 6 und 7, dadurch gekennzeichnet, daß in einem Behälterstopfen vorbereitet wird:

| | |
|---|---|
| - Eisen(II)-Gluconat | 200 - 1600 mg |
| - Dimeticon | 10 - 80 mg |

und in dem kleinen Eindosisfläschchen:

| | |
|---|---|
| - Fructose | 1000 - 6000 mg |
| - Sorbitose | 300 - 2000 mg |
| - Glycerin | 300 - 2000 mg |
| - Methylparaben | 10 - 20 mg |
| - Propylparaben | 2 - 4 mg |
| - Fruchtaromen | 10 - 50 mg |
| - Citronensäure | q.s.p. pH 3 - 6 |
| - Reinwasser | q.s.p. 10 - 20 g |

9. Verfahren nach Anspruch 8, dadurch gekennzeichnet, daß die nachstehende Rezeptur in dem Behälterstopfen zubereitet wird:

| | |
|---|---|
| - Eisen(II)-Gluconat | 300 mg |
| - Dimeticon | 15 mg |

und die nachstehende Rezeptur in dem kleinen Eindosisfläschchen zubereitet wird:

| | |
|---|---|
| - Fructose | 3000 mg |
| - Sorbitose | 750 mg |
| - Glycerin | 750 mg |
| - Methylparaben | 15 mg |
| - Propylparaben | 3 mg |
| - Fruchtaroma | 20 mg |
| - Citronensäure | q.s.p. pH 4,5 |
| - Reinwasser | q.s.p. 15 g |

Patentansprüche für folgende Vertragsstaaten : AT,ES,GR

1. Verfahren zur Herstellung eines Arzneimittelprodukts für orale Verabreichung (Sirup), bestehend aus einem therapeutisch nutzbaren Eisensalz mit zweiwertigem Eisen, dadurch gekennzeichnet, daß in kleinen Eindosisfläschchen in geeigneter Weise ein mit Süßstoffen und Aromastoffen versetzter wäßriger Träger zubereitet wird, und daß das Eisen(II)-Salz in fester Form in luftdicht verschlossene Behälterstopfen gegeben wird.

2. Verfahren nach Anspruch 1, dadurch gekennzeichnet, daß das Eisen(II)-Salz mit Dimeticon angereichertes Eisen(II)-Gluconat ist.

3. Verfahren nach den Ansprüchen 1 und 2, dadurch gekennzeichnet, daß in einen Behälterstopfen gegeben wird:

- Eisen(II)-Gluconat                    200 - 1600 mg
- Dimeticon                                   10 - 80 mg

und in das kleine Eindosisfläschchen:

- Fructose                          1000 - 6000 mg
- Sorbitose                        300 - 2000 mg
- Glycerin                         300 - 2000 mg
- Methylparaben                  10 - 20 mg
- Propylparaben                   2 - 4 mg
- Fruchtaromen                   10 - 50 mg
- Citronensäure       q.s.p.    pH   3 - 6
- Reinwasser               q.s.p.   10 - 20 g

4. Verfahren nach Anspruch 3, dadurch gekennzeichnet, daß die nachstehende Rezeptur in den Behälterstopfen gegeben wird:

- Eisen(II)-Gluconat                  300 mg
- Dimeticon                                15 mg

und die nachstehende Rezeptur in das kleine Eindosisfläschchen gegeben wird:

- Fructose                        3000 mg
- Sorbitose                        750 mg
- Glycerin                         750 mg
- Methylparaben                  15 mg
- Propylparaben                   3 mg
- Fruchtaroma                    20 mg
- Citronensäure       q.s.p.    pH   4,5
- Reinwasser               q.s.p.   15 g